# EUROPÄISCHE PATENTANMELDUNG

(11) **EP 0 676 233 A1**
(43) Veröffentlichungstag der Anmeldung: **11.10.1995**
(21) Anmeldenummer: 95101677.3
(22) Anmeldetag: 08.02.1995
(51) Int. Cl.: B01D 61/12, B01D 65/02, B01D 65/10

(54) **Membranüberwachung beim Umkehrosmoseverfahren**

(30) Priorität: 09.04.1994 DE 4412284
(71) Anmelder: STEAG Aktiengesellschaft, D-45128 Essen (DE)
(72) Erfinder: Maxein, Hans-Georg, Dipl.-Ing., D-45475 Mülheim (DE)

(57) **Zusammenfassung**

Rohwasser (1) wird zur Reinigung durch ein Druckrohr (10) mit fünf in Reihe geschalteten Hauptmembranen (11-15) geleitet. Vor dem Druckrohr wird ein Teilstrom (5) des Rohwassers (1) abgezweigt und mehreren parallel geschalteten Sätzen aus jeweils fünf Referenzmembranen (111-151N,112-15N,...,11M-15M) zugeführt. Diese Referenzmembranen werden unter den gleichen Betriebsbedingungen wie die zugehörigen Hauptmembranen betrieben. Zur Überwachung des sich auf der Hauptmembran ablagernden Biofilms werden in zeitlichen Abständen einzelne Sätze von Referenzmembranen entnommen und analysiert. Aus den Anlaysenergebnissen wird der Zeitpunkt für den nächsten Reinigungszyklus bestimmt. Die Reinigungsart kann speziell auf die ermittelte Foulingart abgestimmt werden.

## Beschreibung

Die Erfindung betrifft ein Umkehrosmoseverfahren zum Aufteilen von Rohwasser in Permeat und Konzentrat an mindestens einer Hauptmembran, die zyklisch gereinigt wird.

An Hauptmembranen, die mit Rohwasser beaufschlagt werden, lagern sich mit der Zeit Stoffe auf der Membranoberfläche ab. Diese Ablagerung von Stoffen des Rohwassers auf Oberflächen wird allgemein als "Fouling" bezeichnet. Durch die Ablagerung nimmt der Membranwiderstand zu, wodurch die Leistungsfähigkeit der Membran und somit die wirtschaftliche Anwendung des Membrantrennverfahrens beeinträchtigt werden. Je nach Art der abgelagerten Stoffe unterscheidet man verschiedene Arten des Fouling: Die Ablagerung von anorganischen Stoffen, deren Löslichkeitsprodukt durch Aufkonzentrierung überschritten wird, bezeichnet man als "Scaling". Werden organische Stoffe adsorbiert, handelt es sich um "Organic Fouling". Bei der Adhäsion von Mikroorganismen spricht man vom "Biofouling"; die Mikroorganismen lagern sich dabei eingebettet in einen Gel aus extra-zellulären Polymeren als Biofilm auf der Membranoberfläche ab. Durch intensive Untersuchung der beiden zuerst genannten Foulingarten, Scaling und Organic Fouling, sind für diese inzwischen wirksame Reinigungsmaßnahmen bekannt. Der Mechanismus des Biofouling ist dagegen bisher weitgehend ungeklärt. Diese Ablagerungsart ist für Hauptmembranen besonders problematisch, da sich die Mikroorganismen am Ablagerungsort komplex vermehren können und deshalb kein unmittelbarer Zusammenhang zwischen der Wachstumsrate der Biofilmdicke und der Mikroorganismenkonzentration im Rohwasser besteht.

Es genügt deshalb nicht, die Konzentration der Mikroorganismen im Rohwasser zu überwachen, um Informationen über die Wachstumsrate der Biofilmdicke auf Membramen zu gewinnen. Die Vermehrung der Mikroorganismen auf Membranen im Rahmen des Biofoulings hängt von einer Vielzahl von Parametern ab, wie Art der Spezies, Nährstoffkonzentration, Oberflächenladung der im Wasser gelösten Stoffe, chemische Zusammensetzung der Membran, Oberflächenladung der Membran, Temperatur und Druck des Rohwassers, Konzentration und Art der anderen gelösten Stoffe usw.

Es ist bislang nicht möglich, die Entwicklung des Biofilms längs der Membranoberfläche zu überwachen, da es sich in der Regel um geschlossene Systeme handelt, in die die Membranen integriert sind. Ein Anstieg der Biofilmschichtdicke auf Hauptmembranen kann daher nur anhand von Veränderungen nicht Biofilm relevanter Meßgrößen festgestellt werden. Beim Foulingprozeß tritt beispielsweise eine erhöhte Druckdifferenz zwischen dem Wasserdruck auf der Rohwasserseite und dem Wasserdruck auf der Permeat-/Konzentratseite auf. Genauso kann Fouling anhand einer Abnahme der Permeatausbeute, die beim Biofouling bis zu 20-30 % abnimmt, festgestellt werden. Auch eine abnehmende Salzrückhaltung durch die Hauptmembran ist ein Indiz für auftretendes Biofouling. Da diese drei Meßgrößenveränderungen jedoch auch bei jeder anderen Art des Foulings auftreten, weiß man zunächst nicht, um welche Art des Foulings es sich handelt und welche Reinigungsmaßnahmen angemessen sind. In der Praxis werden in der Regel beim Auftreten einer Parameterveränderung, die auf einen Foulingprozess schließen läßt, zunächst Maßnahmen gegen abiotische Ursachen gerichtet, da das Biofouling seltener auftritt. Erst wenn diese Maßnahmen fehlgeschlagen sind, werden Maßnahmen gegen die Beseitigung eines Biofilms, beispielsweise durch Reinigung mit mechanischen und/oder mit chemischen Mitteln getroffen. Die Reinigung der Membran vom Biofouling findet jedoch in jedem Fall - auch bei sofortiger Einleitung der Maßnahmen nach beispielsweise einem Druckanstieg - zu einem Zeitpunkt statt, zu dem das Biofouling schon stark fortgeschritten bzw. irreversible Verblockung bereits eingetreten ist. Die charakteristischen Meßgrößen sprechen erst sehr spät an und erlauben keine Aussage über das Anfangsstadium des Biofouling.

Die Nachteile einer so späten Biofoulingbekämpfung bestehen darin, daß die Membran vor der Biofoulingerkennung über einen beträchtlichen Zeitraum unwirtschaftlich betrieben worden ist: Die Trennleistung, insbesondere die Salzrückhaltung der Membran läßt nach, wodurch die Qualität des gereinigten Rohwassers abnimmt. Außerdem verringert sich die Permeatausbeute, d.h. die Quantität des gereinigten Rohwassers. Zusätzlich ist ein erhöhter Energieaufwand zur Erzeugung der erhöhten Druckdifferenz erforderlich, die für den Betrieb der schon vom Biofouling betroffenen Hauptmembran aufgebracht werden muß. Ein weiterer Nachteil besteht darin, daß die Wirksamkeit der Reinigungsmaßnahmen bei Anwendung auf eine mit einem Biofilm überzogene Membran mit der Zunahme des Biofoulings abnimmt, d.h. je später die Reinigung der Hauptmembran einsetzt, desto geringer ist der Reinigungseffekt. Bei mehrfacher Reinigung nimmt auch die jeweilige Restverblockung zu. Die Lebensdauer der Membran wird daher erheblich reduziert, wodurch beträchtliche zusätzliche Anschaffungskosten anfallen.

Zur Vermeidung dieser Nachteile, die durch die fehlende Überwachungsmöglichkeit des Membranzustandes entstehen, kann die Membran vorsorglich alternativ in gleich bleibenden Zeitabständen gereinigt werden. Diese Zeitabstände werden allein auf nicht-meßbaren Erfahrungswerten basierend so kurz gewählt, daß ein Druckanstieg an der Membran erst gar nicht auftritt. Auf diese Weise werden zwar die Trennleistung und die Permeatausbeute weitgehendst gewahrt, jedoch sind die durch regelmäßige Spülwasserzyklen entstehenden Betriebskosten, insbesondere die zusätzlichen Abwasserkosten die Abwasserbelastungen und die Verluste durch Stillstandszeiten erheblich.

Aufgabe der Erfindung ist es deshalb, eine einfache Möglichkeit zur zuverlässigeren Überwachung des Zustandes der Hauptmembran zu schaffen.

Diese Aufgabe wird erfindungsgemäß dadurch gelöst, daß
a) ein Teilstrom des Rohwassers und ggf. des Permeats abgezweigt und jeder Teilstrom mehreren parallel geschalteten Referenzmembranen zugeführt wird,
b) an den Referenzmembranen im wesentlichen die Betriebsparameter der Hauptmembran eingestellt werden,
c) in zeitlichen Abständen Proben der Referenzmembranen entnommen und analysiert werden und
d) aus den Analysenergebnissen der Zeitpunkt für den nächsten Reinigungszyklus bestimmt wird.

Als Referenzmembranen können dabei nicht nur identische Membranen der gleichen Zusammensetzung, mikroskopischen Struktur und Formgebung wie die Hauptmembran, sondern auch insbesondere einfacher aufgebaute Membranen mit ähnlichen Trenneigenschaften verwendet werden. Zur Untersuchung des Teilstromes genügen dabei relative kleine und somit kostengünstige Referenzmembranen. Durch die Parallelbeaufschlagung der Referenzmembranen können Proben von Referenzmembranen oder ganze Referenzmembranen nacheinander ohne Auswirkung auf die Betriebsparameter der verbleibenden Referenzmembranen entnommen werden. Diese können dann zu späteren Zeitpunkten entnommen werden und Aufschluß über den zeitlichen Verlauf des Foulingprozesses geben.

Bei Entnahme einer Referenzmembran können an dieser Labormessungen zur Analyse des Membranbelages durchgeführt werden. Es handelt sich bei diesen Analysenmethoden hauptsächlich um spektroskopische Verfahren bzw. Röntgenfluoreszenzverfahren, bei denen von der Referenzmembran bzw. Probe ein Probenstück entnommen, geeignet präpariert und untersucht wird. Die Referenzmembran wird dabei in den meisten Fällen zerstört. Genauso können aber mikroskopische Verfahren angewendet werden, bei denen die Referenzmembran nicht beschädigt wird, so daß sie zu einem Zeitpunkt an dem reproduzierbare Betriebsparameter an der Hauptmembran vorliegen, wieder als Referenzmembran eingebaut werden kann. Reproduzierbare Betriebsparameter an der Hauptmembran liegen beispielsweise nach einer Membranreinigung vor oder dann, wenn anhand einer zweiten entnommenen Referenzmembran festgestellt wird, daß seit der Entnahme der ersten Referenzmembran der Membranbelag keine Veränderung erfahren hat.

Die Entnahme von Referenzproben hat den erheblichen Vorteil, daß genaue Aussagen über die Art und die Menge des Membranbelages gemacht werden können. Dabei ist es nicht unbedingt erforderlich, daß die Dicke des Belages auf der Referenzmembran der Dicke des Belages auf der Hauptmembran entspricht, sondern es muß nur ein reproduzierbarer Zusammenhang zwischen Referenzmembranzustand und Hauptmembranzustand bestehen. Dieser Zusammenhang kann ggf. in Eichmessungen genau ermittelt werden.

Mit dem erfindungsgemäßen Verfahren ist es also möglich, anhand des Zustandes der Referenzmembran den Zustand der Hauptmembran in regelmäßigen Abständen bei nur geringen Kosten zu überwachen. Die Reinigung der Hauptmembran kann auf diese Weise sowohl zu einem geeigneten Zeitpunkt als auch mit geeigneten Maßnahmen durchgeführt werden. Die Kosten, die durch diese speziell an den Hauptmembranzustand angepaßten Reinigungsmaßnahmen eingespart werden, sind enorm. Die Lebensdauer der Hauptmembran kann beträchtlich verlängert werden, und zudem können Qualität und Quantität des Permeats auf einem hohen Pegel gehalten werden.

In Weiterbildung der Erfindung wird vorgeschlagen, daß als Proben Teilstücke der Referenzmembranen oder des Belages der Referenzmembranen entnommen werden, wobei die Beaufschlagung der Reststücke im wesentlichen ununterbrochen fortgesetzt wird. Auf diese Weise können die Betriebskosten für die Überwachung der Hauptmembran weiter reduziert werden, da eine Referenzmembran für mehrere Nachweisanalysen genutzt werden kann.

In der Praxis wird Rohwasser meist in mehreren Reinigungsstufen gereinigt. Vorteilhafterweise wird deshalb bei Verwendung mehrerer in Reihe geschalteter Hauptmembranen ein Teilstrom des Rohwassers bzw. Permeats zwischen aufeinanderfolgenden Hauptmembranen abgezweigt und mehreren parallel geschalteten Referenzmembranen zugeführt. Es ist so möglich, jede Hauptmembran unabhängig von den anderen Reinigungsstufen zu überwachen, da durch erneutes Abzweigen des Rohwassers bzw. Permeats vor der jeweiligen Hauptmembran an den zugehörigen Referenzmembranen im wesentlichen die gleichen Betriebsparameter wie an der jeweiligen Hauptmembran eingestellt werden.

Ein bevorzugtes Ausführungsbeispiel ist dadurch gekennzeichnet, daß bei Verwendung mehrerer in Reihe geschalteter Hauptmembranen zu wenigstens einem Teil der parallel geschalteten Referenzmembranen weitere Referenzmembranen in Reihe geschaltet sind, wobei jeweils die Gesamtzahl der in Reihe geschalteten Referenzmembranen der Anzahl der in Reihe geschalteten Hauptmembranen entspricht. Diese Ausführungsform kann insbesondere bei in Reihe geschalteten Hauptmembranen eingesetzt werden, die nicht frei zugänglich sind, weil sie beispielsweise als einzelne Module in ein geschlossenes Druckrohr integriert sind. Dann genügt es, vor der ersten Hauptmembran einen Teilstrom zu entnehmen und diesen durch mehrere parallel geschaltete Sätze aus in Reihe geschalteten Referenzmembranen zu leiten. Da die Anzahl der jeweils in Reihe geschalteten Referenzmembranen der Anzahl der Hauptmembranen entspricht, kann der Zustand der n-ten Hauptmembran anhand des Zustandes der n-ten Referenzmembranen in den parallel geschalteten Referenzmembransätzen überwacht werden. Dabei ist es nicht zwingend, daß bei jeder Probenentnahme ein ganzer Satz von Referenzmembranen entnommen wird, sondern es können zusätzlich einzelne Referenzmembrane oder Sätze aus wenigeren Referenzmembranen parallel zu dem Teilstrom geschaltet werden, die dann zur Überwachung der Hauptmembranzustände zunächst entnommen werden. Wird zu dem ersten Überwachungszeitpunkt nach einer Membranreinigung beispielsweise nur eine einzelne Referenzmembran entnommen und an dieser noch kein Fouling festgestellt, so kann die nächste Kontrollmessung abgewartet werden. Wird jedoch zu einem Zeitpunkt an einer einzelnen Referenzmembran ein kritischer Belag festgestellt, so kann zunächst zur genauen Analyse ein Satz der in Reihe geschalteten Referenzmembranen entnommen werden. Anhand dieses Satzes kann dann genau festgestellt werden, welche Hauptmembran sich in einem kritischen Zustand befindet. Die Anzahl der zu untersuchenden und ggf. zerstörten Referenzmembranen wird somit optimiert.

Im folgenden wird die Erfindung anhand eines in der Zeichung dargestellten Ausführungsbeispiels näher erläutert. In der Zeichnung zeigt:

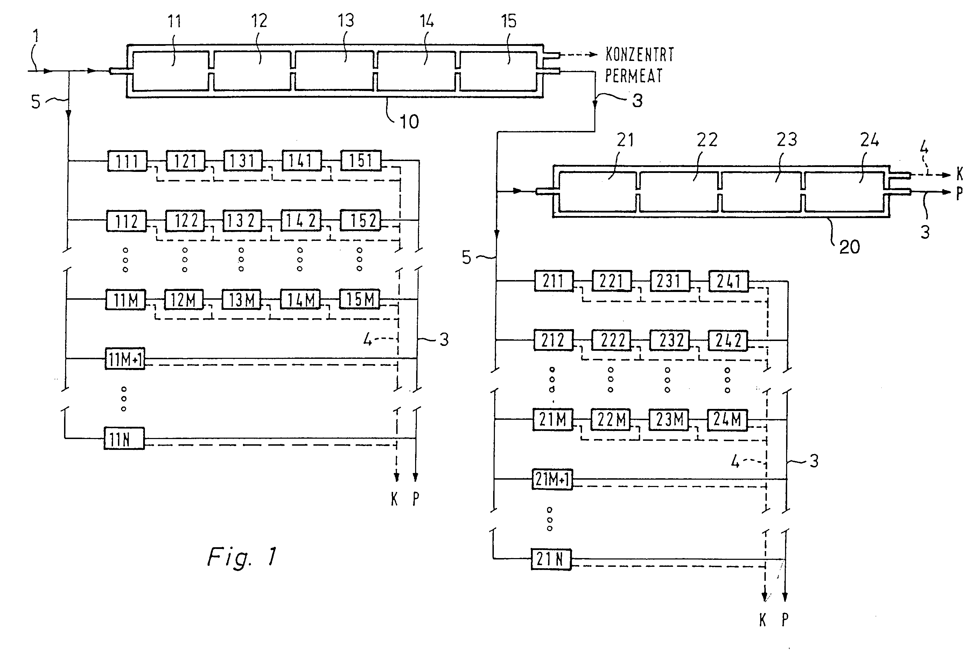

- Fig. 1: ein schematisches Schaltbild der Haupt- und Referenzmembranen bei der überwachten Rohwasser reinigung.

Nach Fig. 1 wird Rohwasser 1 einem Druckrohr 10 zugeführt, welches fünf Wickelmodule 11-15 als Hauptmembrane enthält. Das Rohwasser wird im Druckrohr 10 teilgereinigt und damit in Permeat 3 und Konzentrat 4 aufgeteilt. Das Permeat 3 gelangt anschließend zu einem zweiten Druckrohr 20, welches mit vier Wickelmodulen 21-24 als Hauptmembranen bestückt ist. Hier erfolgt eine weitere Reinigung, nämlich erneut eine Aufteilung in Permeat 3 und Konzentrat 4.

Von dem Rohwasser 1 wird vor Eintritt in das erste Druckrohr 10 ein Teilstrom 5 abgeführt. Mit diesem Teilstrom 5 werden parallel Referenzmembranen in den Modulen 111, 112, 113 ... 11N beaufschlagt. Diese Module trennen unter den gleichen Betriebsbedingungen wie das Modul 11 Permeat und Konzentrat. Das Konzentrat wird zusammengeführt und abgeleitet. Das Permeat wird von den Referenzmodulen 11M+1 ... 11N ebenfalls unmittelbar abgeführt, jedoch wird das Permeat von den Referenzmodulen 111 ... 11M durch weitere in Reihe geschaltete Sätze von Referenzmodulen 121 ... 151, 122 ... 152, usw. bis 12M ... 15M geleitet. In den verschiedenen Sätzen der in Reihe geschalteten Referenzmodule entsprechen die Betriebsparameter des n-ten beaufschlagten Referenzmoduls jeweils den Betriebsparametern des n-ten beaufschlagten Hauptmoduls.

Vor Eintritt des Permeats 3 aus dem ersten Druckrohr 10 in das zweite Druckrohr (20) wird wiederum ein Teilstrom 5 dem vorgereinigten Permeat entnommen. Mit diesem Teilstrom werden parallel die Referenzmodule 211, 212, 213 ... 21N beaufschlagt, die unter den gleichen Betriebsbedingungen wie der Hauptmodul 21 Konzentrat und Permeat voneinander trennen. Das Permeat wird auch bei diesem zweiten Druckrohr von einem Teil der Referenzmodule, nämlich 21M+1 ... 21N direkt abgeführt, jedoch wird das Permeat bei dem anderen Teil der Referenzmodule, nämlich den Modulen 211 ... 21M, in weitere in Reihe geschaltete Referenzmodule 222 ... 242 usw. bis 22M ... 24M geleitet.

Zur Analyse der Hauptmembranen in den Hauptmodulen werden dann in regelmäßigen Abständen einzelne oder einzelne Sätze der parallel geschalteten Referenzmembranen aus den Referenzmodulen entnommen. Diese Referenzmembranen können dann genau analysiert werden und geben nicht nur Aufschluß über die Dicke des Membranbelages sondern auch über die Art des Foulings und somit über das anzuwendende Reinigungsverfahren. Wird festgestellt, daß die Hauptmembranen in den Hauptmodulen 11-15 bzw. 21-24 gereinigt werden müssen, so werden in analoger Weise die Referenzmembranen mitgereinigt. Besonders wirtschaftlich ist es, unmittelbar nach einer Reinigung jeweils nur einzelne Referenzmembranen 11M+1...11N bzw. 21M+1... 21N zu entnehmen. Erst bei einer kritische Veränderung des Membranzustandes kann dann eine genaue Analyse mit Hilfe eines der noch betriebenen vollständigen Sätze von Referenzmembranen (111 ... 151 usw. bis 11M ... 15M bzw. 211 ... 241 usw. bis 21M .. 24M) erfolgen.

Mit dem erfindungsgemäßen Verfahren gelingt es also, den Abscheidungsprozeß auf Membranen mit Hilfe von parallel geschalteten Referenzmembranen in beliebigen zeitlichen Abständen zu kontrollieren und somit das Anwachsen von insbesondere Biofilmen zu überwachen. Ein wirtschaftlicher Betrieb der Hauptmembran und eine hohe Qualität des Permeats können so bei verringertem Reinigungsaufwand und entsprechend reduzierter Umweltbelastung gewährleistet werden.

Im Rahmen des Erfindungsgedankens sind viele Variationsmöglichkeiten gegeben. Insbesondere kann jede beliebige Anzahl von Referenzmembranen in Reihe geschaltet werden. Genauso ist es denkbar, daß nach jedem Reinigungsvorgang neue Referenzmodule verwendet werden, anstatt daß die Referenzmodule mitgereinigt werden. Auch die Art der Referenzmembran kann sich von der Hauptmembran beliebig unterscheiden, wichtig ist nur, daß es ein reproduzierbarer Zusammenhang zwischen der Beaufschlagung der Hauptmembranen und der Beaufschlagung der Referenzmembranen gibt.

## Patentansprüche

1. Umkehrosmoseverfahren zum Aufteilen von Rohwasser (1) in Permeat (3) und Konzentrat (4) an mindestens einer Hauptmembran (11-15,21-24), die zyklisch gereinigt wird,
**dadurch gekennzeichnet,** daß
a) ein Teilstrom (5) des Rohwassers (1) und ggf. des Permeats (3) abgezweigt und jeder Teilstrom mehreren parallel geschalteten Referenzmembranen (111-11N, 211-21N) zugeführt wird,
b) an den Referenzmembranen (111-11N, 211-21N) im wesentlichen die Betriebsparameter der Hauptmembran eingestellt werden,
c) in zeitlichen Abständen Proben der Referenzmembranen (111-11N, 211-21N) entnommen und analysiert werden und
d) aus den Analysenergebnissen der Zeitpunkt für den nächsten Reinigungszyklus bestimmt wird.

2. Verfahren nach Anspruch 1, dadurch gekennzeichnet, daß als Proben Teilstücke der Referenzmembranen (111-15N,211-24N) oder des Belages der Referenzmembranen (111-15N,211-24N) entnommen werden, wobei die Beaufschlagung der Reststücke im wesentlichen ununterbrochen fortgesetzt wird.

3. Verfahren nach Anspruch 1 oder 2, dadurch gekennzeichnet, daß bei Verwendung mehrerer in Reihe geschalteter Hauptmembranen (11-15,21-24) ein Teilstrom (5) des Rohwassers (1) bzw. Permeats (3) zwischen aufeinanderfolgenden Hauptmembranen abgezweigt und mehreren parallel geschalteten Referenzmembranen (111-15N,211-24N) zugeführt wird.

4. Verfahren nach Anspruch 1 oder 2, dadurch gekennzeichnet, daß bei Verwendung mehrerer in Reihe geschalteter Hauptmembranen (11-15,21-24) zu wenigstens einem Teil der parallel geschalteten Referenzmembranen (111-11N, 211-21N) weitere Referenzmembranen (121-15N, 221-24N) in Reihe geschaltet sind, wobei jeweils die Gesamtzahl der in Reihe geschalteten Referenzmembranen der Anzahl der in Reihe geschalteten Hauptmembranen entspricht.
